(19) Europäisches Patentamt
European Patent Office
Office européen des brevets

(11) Veröffentlichungsnummer: **0 194 543**
**B1**

(12) # EUROPÄISCHE PATENTSCHRIFT

(45) Veröffentlichungstag der Patentschrift: **10.05.89**

(21) Anmeldenummer: **86102764.7**

(22) Anmeldetag: **03.03.86**

(51) Int. Cl.⁴: **C 07 D 213/79**, C 07 D 213/84, C 07 D 213/50, C 07 D 213/46, C 07 D 213/48, C 07 D 213/80, C 07 D 213/85, B 05 D 5/12, H 01 B 1/12, C 07 C 121/48

(54) **Schmelzbare, elektrisch leitfähige TCNQ-Komplexe.**

(30) Priorität: **14.03.85 DE 3509070**

(43) Veröffentlichungstag der Anmeldung: **17.09.86 Patentblatt 86/38**

(45) Bekanntmachung des Hinweises auf die Patenterteilung: **10.05.89 Patentblatt 89/19**

(84) Benannte Vertragsstaaten: **CH DE FR GB IT LI**

(56) Entgegenhaltungen:
**DE-A-2 329 492**
**US-A-3 162 641**

**JOURNAL OF THE AM. CHEM. SOCIETY, Band 84, 1962, Easton, Pa., L.R. MELBY et al. "Substituted Quino-dimethans", pp. 3374-3387**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Jonas, Friedrich, Dr., Krugenofen 15, D-5100 Aachen (DE)**
Erfinder: **Hocker, Jürgen, Dr., Eichenweg 6, D-5060 Bergisch- Gladbach 2 (DE)**

**EP 0 194 543 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Komplexsalze aus dem 7.7.8.8-Tetracyano-p-chinodimethananion $(TCNQ)^{\ominus}$,

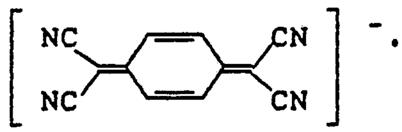

neutralem 7.7.8.8-Tetracyano-p-chinodimethan (TCNQ) und anorganischen oder organischen Kationen sind als elektrisch leitende Verbindungen bekannt und können durch Umsetzung von TCNQ mit organischen Kationjodiden hergestellt werden [J. Am. Chem. Soc. 84, 3374 - 3378 (1962)], z. B. gemäß

$$4\,TCNQ + 3M^+J^- \rightarrow 2\,(M^+ \cdot TCNQ^- \cdot TCNQ) + M^+J_3^-$$

Reaktionsschema I.

worin M z. B. ein N-Alkylchinolinium-Kation ist.

Hierbei wird ein TCNQ-Molekül durch Jodid zum TCNQ Radikal-Anion unter Freisetzung von Jod reduziert.

Ein anderes Verfahren besteht in der Umsetzung von stickstoffhaltigen Heteroaromaten oder tertiären Aminen mit $H_2TCNQ$ und TCNQ, z. B. gemäß

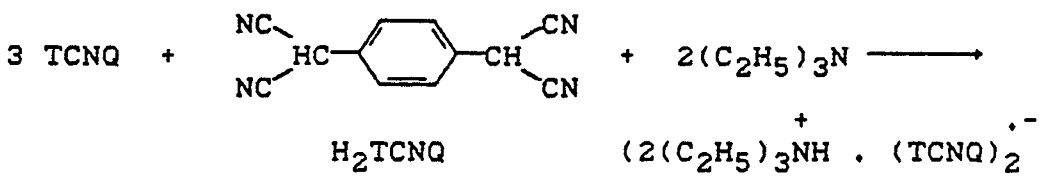

Reaktionsschema II

Die Verarbeitung dieser Komplexe bei Temperaturen oberhalb ihres Schmelzpunktes bereitet Schwierigkeiten, da die bisher bekannten Verbindungen sich bereits am Schmelzpunkt oder wenig oberhalb des Schmelzpunktes zersetzen (s. Tab. 1).

Ferner ist aus der DE-A-2 329 492 bekannt, die Wärmestabilität von Pyridinum-TCNQ-Komplexen durch Substitution des Pyridin-Stickstoffes zu erhöhen. Aber auch die Wärmestabilität der in der DE-A-2 329 492 beschriebenen N-substituierten Pyridinium-TCNQ reicht für eine Verarbeitbarkeit dieser Komplexverbindungen aus ihren Schmelzen nicht aus.

Es wurde gefunden, daß Komplexe aus TCNQ und speziellen organischen Pyridiniumionen längere Zeit im geschmolzenen Zustand gehalten werden können, ohne daß ihre elektrische Leitfähigkeit nach dem Erkalten wesentlich vermindert ist.

Gegenstand der Erfindung sind daher TCNQ-Komplexe der allgemeinen Formel I

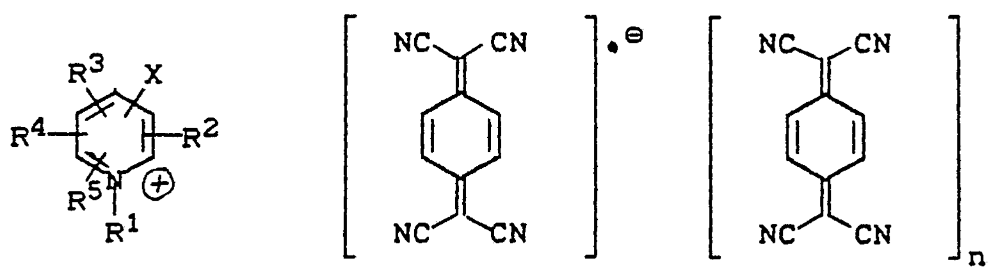

worin

$2 \geqslant n \geqslant 0$,

$R^1$, $R^2$, $R^3$, $R^4$, $R^5$ gleich oder verschieden, Wasserstoff oder einen geradkettigen oder verzweigten Alkylrest mit 1 bis 30 C-Atomen, einen Cycloalkylrest mit 5 bis 12 C-Atomen, einen Arylrest mit 6 bis 14 C-Atomen oder einen heterocyclischen Rest mit 4 bis 12 C-Atomen bedeuten, mit der Maßgabe daß $R^1 \neq$ Wasserstoff ist und

X für einen Acylrest der Formeln

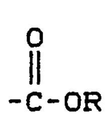 oder 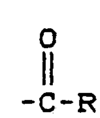

steht, in denen R die für $R^1$ angegebene Bedeutung hat.

Als Beispiele für $R^1$ und R seien formelmäßig angegeben:

$CH_3-CH_2-CH_2-$ $CH_3-CH_2-CH_2-CH_2-$ $CH_3-CH_2-CH-$
$CH_3$

$CH_3$
CH-
$CH_3$

$CH_3-CH_2-$

$CH_3-$

$CH_3-CH_2-CH_2-CH_2-CH_2-$
$CH_3-CH_2-CH_2-CH_2-CH_2-CH_2-$
$CH_3-CH_2-CH_2-CH_2-CH_2-CH_2-CH_2-$
$CH_3-CH_2-CH_2-CH_2-CH_2-CH_2-CH_2-CH_2-CH_2-$
$CH_3-CH_2-CH_2-CH_2-CH-CH_2-$
$C_2H_5$

$CH_2=CH-CH_2-$

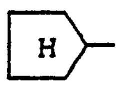 , 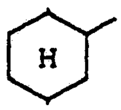 , 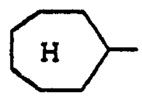 .

Besonders bevorzugt sind Verbindungen der Formel I, in denen $R^2$, $R^3$, $R^4$ und $R^5$ gleich Wasserstoff und $R^1$, R gleich oder verschieden $C_1$-$C_{12}$ Alkyl sind.

Die Komplexe der Formel I können nach Reaktionsschema I, wie in J. Am. Chem. Soc. 84, 3374 - 3387 (1962) beschrieben, hergestellt werden.

Die Herstellung der erfindungsgemäßen Verbindungen erfolgt vorteilhaft durch Umsetzung von Lösungen substituierter, organischer Pyridiniumjodide mit einer Lösung von TCNQ in organischen Lösungsmitteln bei Temperaturen unterhalb 150° C.

Geeignete organische Lösungsmittel sind z. B. Halogenkohlenwasserstoffe, z. B. Methylenchlorid, Chloroform, Tetrachlorkohlenstoff, 1,2-Dichlorethan, 1,1,2-Trichlorethan; Acetonitril; Alkohole, z. B. Methanol, Ethanol, Isopropanol; aliphatische Ketone, z. B. Aceton, Methylethylketon; acyclische und cyclische Ether, z. B. Diethylether, Tetrahydrofuran.

Die Reaktionspartner werden im Verhältnis 1 Mol TCNQ zu 0,5 bis 1,5 Mol Pyridiniumjodid eingesetzt.

Aufgrund ihres günstigen Schmelz- und Zersetzungsverhaltens können die Verbindungen der Formel I zur Herstellung elektrisch leitender Überzüge auf Substraten durch Aufschmelzen verwendet werden.

In vorteilhafter Weise können auch Gemische verschiedener TCNQ-Komplexe, die mindestens eine Verbindung der Formel I enthalten, aus der Schmelze zu leitenden Überzügen verarbeitet werden. Diese Überzüge können gegebenenfalls auch stabilisierende, die Haftung verbessernde bzw. farbgebende, oder den Schmelzpunkt erniedrigende Zusatze enthalten.

Als geeignete Substrate seien genannt: Glas, Metalle, Metalloxide, organische Polymere.

Das Beschichten der Substrate kann in der Weise erfolgen, daß die Substrate auf Temperaturen oberhalb des Schmelzpunktes der TCNQ-Komplexe der Formel I aufgeheizt werden und anschließend die festen TCNQ-Komplexe auf die Substratoberfläche gebracht werden.

Das Aufschmelzen der TCNQ-Komplexe kann auch unter Schutzgasen wie z. B. Wasserstoff, Stickstoff, Argon oder Helium bzw. im Vakuum durchgeführt werden.

Bei einem anderen Verfahren geht man so vor, daß die TCNQ-Komplexe der Formel I bei Raumtemperatur auf die zu beschichtenden Substrate aufgebracht und anschließend in einem vorgeheizten Ofen aufgeschmolzen werden.

Weiterhin besteht die Möglichkeit, die Substrate durch Tauchen in eine Schmelze der Komplexe der Formel I zu beschichten.

Diese Verfahren führen zu elektrisch leitfähigen, gut haftenden Überzügen.

Die so hergestellten Überzüge finden Verwendung in der Elektronikindustrie.

**Beispiel 1**

20,4 g TCNQ in 1000 ml Acetonitril werden bei Rückfluß mit einer 70° C heißen Lösung aus 22,0 g 1-Methyl-4-carbethoxy-pyridiniumjodid in 100 ml Acetonitril versetzt und 10 Minuten bei Rückfluß gerührt. Anschließend wird abgekühlt und der über Nacht auskristallisierte Niederschlag abgesaugt und getrocknet. Bei gut löslichen

Komplexen kann die Ausbeute durch Einengen der Mutterlauge erhöht werden.
Ausbeute: 24,4 g (85 %, d. Th.)
Komplex der Zusammensetzung:

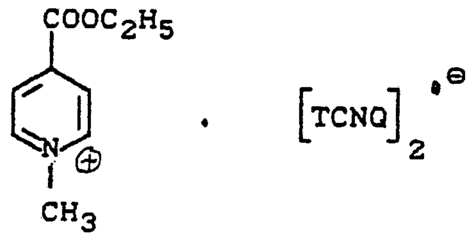

**Beispiel 2**

Wie in Beispiel 1 beschrieben, werden 10,2 g TCNQ in 500 ml Acetonitril und 11 g 1-Methyl-2-carbethoxy-pyridiniumjodid in 100 ml Acetonitril zur Reaktion gebracht und aufgearbeitet.
Ausbeute: 12,6 g (88 % d. Th.)
Komplex der Zusammensetzung:

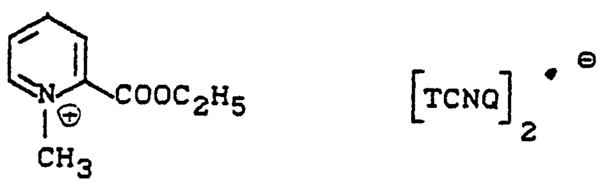

**Beispiel 3**

Wie in Beispiel 1 beschrieben, wenden 10,2 g TCNQ in 400 ml Acetonitril und 9,9 g 1-Methyl-4-acetyl-pyridiniumjodid in 100 ml Acetonitril zur Reaktion gebracht und aufgearbeitet.
Ausbeute: 11,7 g (86 %, d. Th.)
Komplex der Zusammensetzung:

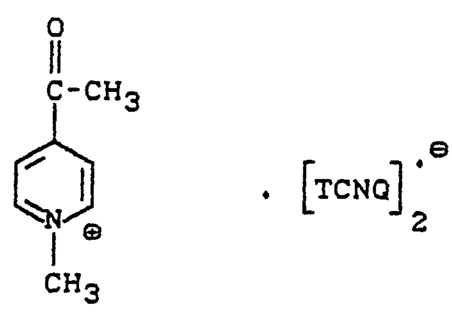

**Beispiel 4**

Die thermische Stabilität der Komplexe nach Beispiel 1 - 3 ist aus Tabelle 1 ersichtlich. Die Komplexe wurden in Glasgefäßen 30 Sekunden bei 260°C aufgeschmolzen und auf Raumtemperatur abgekühlt. Die Leitfähigkeit der reinen Komplexe $\delta°$ vor dem Schmelzen bzw. der erstarrten Schmelze $\delta^s$ wurden mittels Vierelektrodenmessung unter Druck (250 kp/cm) bestimmt.

**Tabelle 1**

| Komplex nach Beispiel | δ° S/cm | δˢ S/cm |
|---|---|---|
| 1 | 1,6 | 0,25 |
| 2 | 0,5 | 0,2 |
| 3 | 0,8 | 0,06 |
| Vergleich: | 0,5 | $5 \cdot 10^{-8}$ |
| Vergleich: | 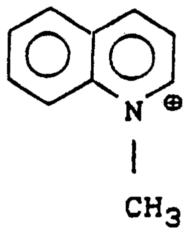 | $[TCNQ]_2^{\ominus}$ |

**Beispiel 5 - 7**

Auf Glasplatten wurden ca. 0,5 mm dicke Schichten der Komplexe 1 - 3 aufgestreut und anschließend im Warmluftofen bei 300° C Lufttemperatur, bis eine homogene Schmelze entstanden war, erhitzt.

In allen Fallen wurden harte, glänzende elektrisch leitfähige Überzüge erhalten.

## Patentansprüche

1. TCNQ-Komplexsalze der allgemeinen Formel I

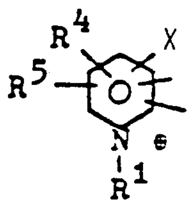 · 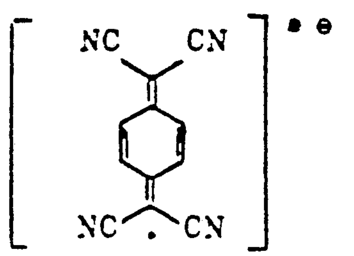 · 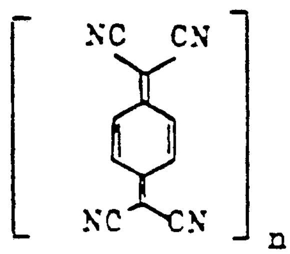 (I)

worin

$2 \geq n \geq 0$,

$R^1$, $R^2$, $R^3$, $R^4$, $R^5$ gleich oder verschieden, Wasserstoff oder einen geradkettigen oder verzweigten Alkylrest mit 1 bis 30 C-Atomen, einen Cycloalkylrest mit 5 bis 12 C-Atomen, einen Arylrest mit 6 bis 14 C-Atomen oder einen heterocyclischen Rest mit 4 bis 12 C-Atomen bedeuten, mit der Maßgabe daß $R^1 \neq$ Wasserstoff ist und

X für einen Acylrest der Formeln

$$-\overset{O}{\overset{\|}{C}}-OR \quad \text{oder} \quad -\overset{O}{\overset{\|}{C}}-R$$

steht, in denen R die für $R^1$ angegebene Bedeutung hat.

2. TCNQ-Komplexsalze gemäß Anspruch 1, dadurch gekennzeichnet, daß in Formel $R^2$, $R^3$, $R^4$ und $R^5$ Wasserstoff sind und $R^1$ und R gleiche oder verschiedene $C_1$-$C_{12}$-Alkylreste bedeuten.

3. Verwendung der Komplexe nach Ansprüchen 1 und 2 zur Herstellung elektrisch leitfähiger Überzüge durch Aufschmelzen auf Substraten.

## Claims

1. TCNQ complex salts of the general formula I

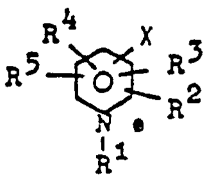 · 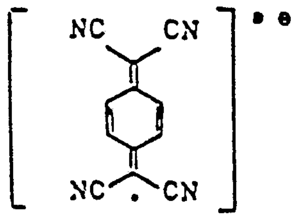 · 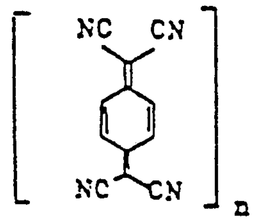 (I)

in which

2 ⩾ n ⩾ 0,

$R^1$, $R^2$, $R^3$, $R^4$, $R^5$ identical or different denote hydrogen or a straight-chain or branched ala radical having 1 to 30 C atoms, a cycloalkyl radical having 5 to 12 C atoms, an aryl radical having 6 to 14 C atoms or a heterocyclic radical having 4 to 12 C atoms, with the proviso that $R^1$ is not hydrogen and

X stands for an acyl radical of the formulae

$$-\overset{O}{\overset{\|}{C}}-OR \quad \text{or} \quad -\overset{O}{\overset{\|}{C}}-R$$

in which R has the meaning given for $R^1$.

2. TCNQ complex salts according to Claim 1, characterized in that in formula I $R^2$, $R^3$, $R^4$ and $R^5$ are hydrogen and $R^1$ and R denote identical or different $C_1$-$C_{12}$-alkyl radicals.

3. Use of the complexes according to Claims 1 and 2 for the preparation of electrically conducting coatings by melting them onto substrates.

**Revendications**

1. Sels complexes de TCNQ de formule générale I

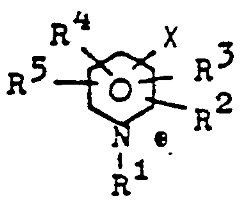 · 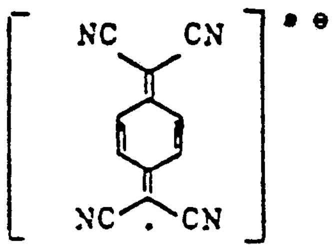 · 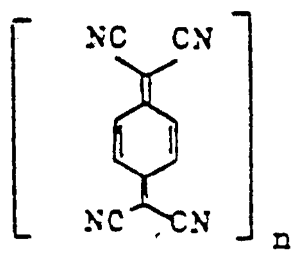 (I)

dans laquelle

n va d'une valeur inférieure ou égale à 2 à une valeur supérieure ou égale à 0,

$R^1$, $R^2$, $R^3$, $R^4$, $R^5$, identiques ou différents, représentent l'hydrogène ou un reste alkyle à chaîne droite ou à chaîne ramifiée ayant 1 à 30 atomes de carbone, un reste cycloalkyle ayant 5 à 12 atomes de carbone, un reste aryle ayant 6 à 14 atomes de carbone ou un reste hétérocyclique ayant 4 à 12 atomes de carbone, sous réserve que $R^1$ soit différent de l'hydrogène et

X représente un reste acyle de formules

$$-\overset{O}{\overset{\|}{C}}-OR \quad \text{ou} \quad -\overset{O}{\overset{\|}{C}}-R$$

dans lesquelles R a la définition indiquée pour $R^1$.

2. Sels complexes de TCNQ suivant la revendication 1, caractérisés en ce que dans la formule I, $R^2$, $R^3$, $R^4$ et $R^5$ représentent l'hydrogène et $R^1$ et R sont des restes alkyle en $C_1$ à $C_{12}$ identiques ou différents.

3. Utilisation des complexes suivant les revendications 1 et 2 pour la production de revêtements conduisant l'électricité par fusion sur des substrats.